Europäisches Patentamt

European Patent Office

Office européen des brevets

(11) Publication number: **0 303 515 B1**

# EUROPEAN PATENT SPECIFICATION

(45) Date of publication of patent specification: **11.11.92**

(51) Int. Cl.⁵: **G01N 33/544**, G01N 33/541, G01N 33/569, G01N 33/571

(21) Application number: **88307523.6**

(22) Date of filing: **12.08.88**

(54) **Improved amphipathic analyte assays using lipophilic surface.**

(30) Priority: **13.08.87 US 85076**

(43) Date of publication of application:
**15.02.89 Bulletin 89/07**

(45) Publication of the grant of the patent:
**11.11.92 Bulletin 92/46**

(84) Designated Contracting States:
**AT BE CH DE ES FR GB GR IT LI LU NL SE**

(56) References cited:
**EP-A- 0 174 106          EP-A- 0 183 383**
**EP-B- 0 098 557          US-A- 3 917 527**
**US-A- 4 241 045          US-A- 4 497 899**

**Proc. Natl. Acad. Sci., 78, pages 3824-28 (1981);**

(73) Proprietor: **SYNBIOTICS CORPORATION**
**11011 Via Frontera**
**San Diego California 92127(US)**

(72) Inventor: **Coleman, Patrick F.**
**12827 Grimsley Avenue**
**Poway, CA 92064(US)**
Inventor: **Andrews, William W.**
**3363 Gregory Street**
**San Diego, CA 92104(US)**
Inventor: **Frazer, John M.**
**321 D Street**
**Chula Vista, CA 92010(US)**
Inventor: **Vodian, Morton A.**
**1051 Arden**
**Encinitas, CA 92024(US)**

(74) Representative: **Sheard, Andrew Gregory et al**
**Kilburn & Strode 30, John Street**
**London WC1N 2DD(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**Description**

The present invention relates to improved assays for amphipathic analytes and in particular to assays for bacterial pathogens.

Lipopolysaccharide (LPS) and lipoprotein antigens are often diagnostic for microorganisms that are disease-causing agents, for example. Chlamydia trachomatis, Chlamydia psittaci, Neisseria gonorrhea, and Escherichia coli. However, analysis of such amphipathic analytes has been difficult because amphipathic molecules are not readily soluble in aqueous medium. To avoid this difficulty, some assays for LPS have avoided detecting the amphipathic analyte and rather analyze for antibodies elicited by infection with the pathogen. See for example Terho et al., J. Med. Microbiol. (1980) 14: 77 which describe analysis of chlamydial infection using a radioimmunoassay (RIA) for Chlamydia-induced antibodies. Such assays are less satisfactory than assays measuring antigen, as antibody is developed several weeks into the course of an infection and can remain elevated for a substantial period of time thereafter.

An alternative approach has been to bind a clinical specimen or a lysed clinical specimen to a solid support. See for example, U.S. Patent Number 4,497,899. However, typically used solid supports such as microtiter plates, test tubes and the like are not uniform with respect to charge density and polarity. Therefore amphipathic molecules can orient in a multiplicity of ways on the surface. Orientation of the antigen has been shown to be critical for monoclonal antibody binding, see Proc. Natl. Acad. Sci. USA Vol.78 pp. 3824-28 (1981), and thus detection of the analyte. Random orientation is particularly severe with samples suspected of containing a bacterial pathogen such as Chlamydia, because the antigen is typically present in small quantities.

Therefore, improved assays for amphipathic analytes which provide for more sensitive detection of such analytes are sought.

Relevant Literature

The following disclosures relate to assays for bacterial pathogens. U.S. Patent No. 4,497,899 (Armstrong) describes a solid phase immunoassay for Chlamydia trachomatis wherein antigen from a lysed specimen is coated on or absorbed on a bare solid support. Terho et al., J. Med. Microbiol. - (1980) 14: 77 describe analysis of chlamydial infection using a radioimmunoassay (RIA) for Chlamydia-induced antibodies. U.S. Patent No. 4,188,371 (Weetall) describes detection of an enzyme released by Neisseria by an immunoradiometric sandwich assay using enzyme-specific antibodies. U.S. Patent No. 4,241,045 (Gaafar) discloses a method for determining the presence of Neisseria gonorrhea wherein the L-antigen is adsorbed onto a solid phase to detect antibodies present in a clinical specimen.

U.S. Patent No. 4,254,082 (Schick) discloses a method and test means for determining a specific binding substance in a liquid sample.

Siliconizing agents are commercially available and have been used to prevent non-specific binding of hydrophilic materials to various surfaces.

SUMMARY OF THE INVENTION

An improved method for assaying an amphipathic analyte is provided in which the lipophilic ends of bipolar molecules are bound to a lipophilic surface of a solid substrate, orienting the polar ends away from the surface. In this way, hydrophilic epitopes of the analyte are available to monoclonal antibodies in the assay medium, resulting in the ability to detect smaller quantities of analyte.

The method comprises incubating an amphipathic analyte dispersed in an aqueous medium with a substantially uniform lipophilic surface for a period of time sufficient for the amphipathic analyte to bind to the lipophilic surface, separating the medium from the lipophilic surface and assaying with monoclonal antibodies for a hydrophilic epitope of the amphipathic analyte on the surface.

BRIEF DESCRIPTION OF THE DRAWINGS

Figure 1 illustrates the random orientation of amphipathic molecules on a typical solid substrate; and
Figure 2 illustrates the orientation of amphipathic molecules on a substantially homogeneous lipophilic surface.

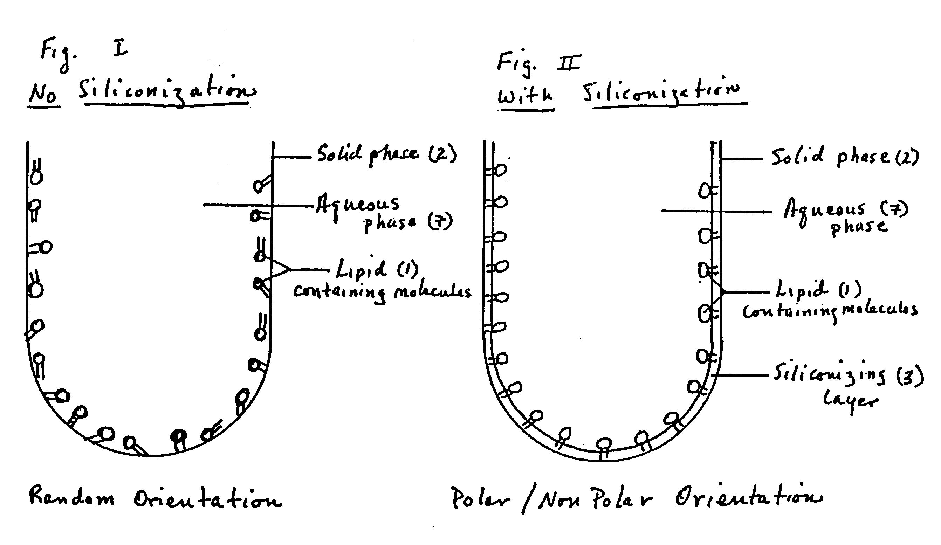

DESCRIPTION OF SPECIFIC EMBODIMENTS

The present invention provides an improved method for assaying an amphipathic analyte. The method relies on binding the analyte to a solid substrate having a substantially uniform lipophilic surface. It has been found that the lipophilic surface allows for the capture and orientation of amphipathic molecules so that their hydrophilic epitopes are optimally presented to the aqueous phase. Ths capture and orientation of the molecules serves a function similar to that provided by monoclonal antibody capture. This allows for optimum binding of monoclonal antibodies directed to hydrophilic epitopes of the amphipathic analyte.

The method comprises incubating an amphipathic analyte comprising a hydrophilic epitope and dispersed in an aqueous medium with a substantially uniform hydrophobic surface of a solid substrate for a period of time sufficient for the analyte to bind to the surface. The medium is separated from the surface, as by washing. An assay for a hydrophilic epitope or the analyte bound to the lipophilic surface is then performed.

Any substantially uniform lipophilic surface coated or resulting from conjugation with lipids, e.g. 6-30 carbon atoms, may be selected for use in the instant method. Alternatively, a substantially uniform lipophilic surface can be achieved by treating most of the solid phase materials which are commonly used for immunoassays with a siliconizing agent. Appropriate siliconizing solutions are alkyl silanes which are prepared in organic solvents. Some preparations (e.g., Aqua-Sil and Silane A-174) are used as dilute aqueous solutions (The terms AQUA-SIL and SILANE A-174 are trade marks). Articles to be siliconized are contacted with the solution and then allowed to dry before use. Other preparations (e.g., Sigmacote) are strictly organic soluble and are not used as aqueous dilutions. (The word SIGMACOTE is a trade mark)
After contacting the article to be siliconized, the excess agent is drained and the article is allowed to dry. The silanes either undergo substitution reactions with reactive groups on the surface that is coated or they react with each other to form a cross-linked film which coats the surface. In either case, the surface is modified with hydrophobic alkyl silanes. For example, suitable commercially available siliconizing agents include Sigmacote (Sigma Chemical Co.), Aqua-Sil (Pierce Chemical Co.) and Silane A-174 (EM Science).

Alkylation with hydrocarbon chains in a manner similar to that used For "reverse phase" chromatographic materials can also be used to provide lipophilic surfaces capable of capturing amphipathic molecules. Commonly used reverse phase chromatographic materials include C-18, C-8, C-4 and Phenyl derivatized porous microspheres. The C values correspond to the length of the hydrophobic aliphatic hydrocarbon chains. Examples of these materials are manufactured commercially by several suppliers and sold under the trademarks of Hypersil (Shandon), Synchropak (Synchrom), Nucleosil, Spherisorb and LiChrosorb.

Virtually all common solid phase materials used in immunoassays (e.g. polystyrene, polyvinyl chloride, latex, rubbers, glass and the like) which can be treated to provide a substantially uniform lipophilic surface, such as materials which can be coated with a layer of a siliconizing agent, can be employed in the present method. In particular, polystyrene, polyvinyl chloride, latex, rubbers, glasses and the like are suitable. The materials can comprise microtiter plates, test tubes, dipsticks and similar solid substrates. Of a wide variety of materials tested, only latex spheres were not as readily suitable for use as the solid phase with certain agents. The spheres tended to clump when coated with a siliconizing agent. Ionic strength adjustments can minimize this problem.

The method is useful for assaying an amphipathic analyte having a hydrophilic epitope for which a monoclonal antibody is specific. Typical of such analytes are lipoproteins and lipopolysaccharides. Of particular interest are lipopolysaccharides or endotoxins that are diagnostic of a pathogen, such as endotoxins produced by Chlamydia trachomatis, Chlamydia psittaci, Neisseria gonorrhea and Escherichia coli.

Samples containing such analytes can comprise serum, plasma, urine, tissue washings, a tissue specimen or a clinical swab. When the analyte is a lipopolysaccharide diagnostic of Chlamydia trachomatis or Chlamydia psittaci, the analytes can be assayed in a purified form (at an appropriate dilution), as a mixture of elementary bodies and infected host cells or as an extract from a swab obtained from an infected individual. When assaying lipopolysaccharide or lipoprotein analytes in a purified form or in a cell-containing sample, the sample can be incubated with an aqueous release buffer prior to assay to optimise the efficiency of the assay.

The release buffer serves a purpose of releasing lipoprotein or lipopolysaccharide antigens from elementary bodies, organisms or cells so that the amphipathic molecule can bind to and orient on the lipophilic surface. Additionally, the presence of host cellular materials such as mucous, epithelial cells and other cellular materials which can potentially interfere with analysis of such sample is substantially reduced in the release buffer-treated sample. Treatment with release buffer results in the amphipathic analyte being dispersed in an aqueous medium which is used in the assay.

The aqueous release buffer will usually have a total ionic strength of 0.1M to 0.5M, optimally about 0.3M and comprises 0.01M to 0.2M, usually 0.05M to 0.15M, sodium phosphate buffer and having NaCl such that the final solution does not exceed a total ionic strength of 0.5M, the concentration of NaCl may therefore be in the range of 0 to 0.3M.

The solution will be buffered to a pH value of from 6.5 to 8.5, usually about a physiological pH, pH 7.4.

The buffer solution will also contain a detergent, usually at a concentration of 0.025 to 0.50% (w/v). (Weight per volume (w/v) means the number of grams of detergent in 100 mL of buffer solution.) A wide variety of detergents may be employed. In particular, ionic, non-ionic, zwitterionic, cholic acid derivatives (e.g., CHAPS) and deoxycholic acid derivatives were tested. Each of the detergents was suitable when tested on purified elementary bodies. However, CHAPS detergent at an optimal concentration for purified elementary bodies proved unsuitable when used with EB-infected cellular material. The detergent apparently extracted material from the cells that competed with the liberated lipopolysaccharide for solid phase binding sites. Preferred detergents are octylglucopyranoside and deoxycholic acid derivatives such as deoxycholate and chenodeoxycholate. Optimal concentration with those detergents is in the range of 0.025 to 0.25% w/v and most preferably 0.1% w/v detergent.

A reducing agent is also present in the release buffer at about 0.01mM to 10mM. N-acetyl cysteine is a preferred reducing agent. Optimal formulations include 1.0mM N-acetyl cysteine. The buffer optionally includes a preservative.

The sample is mixed with release buffer to provide an analyte dispersed in an aqueous medium. If the sample is presented as an aqueous suspension, antigen solublization occurs during the incubation with the solid phase. If the sample is presented on a swab, the swab is vortexed vigorously for one minute in a tube in release buffer and then allowed to stand for 10-15 min. Thereafter, the swab is vortexed for an additional 15 seconds and the extract is expelled from the swab into the tube. A portion of the extract is then incubated with the solid phase. The analyte dispersed in an aqueous medium is incubated with the lipophilic surface, such as a siliconized solid substrate for a period or time sufficient for the analyte to bind to the surface. With typical specimens such as patient swabs the incubation can be at about 37° C. for at least about an hour to overnight, about 18 hours. Those incubation times result in from about 70 to about 100% of the available antigen binding to the surface. Times that provide an equivalent amount of binding can be determined for room temperature or other incubation temperatures.

Following the incubation, the aqueous medium is separated from the surface, as by aspiration. Usually, the surface is washed to ensure removal of non-bound materials. The washing step is usually performed with a wash buffer. The buffer can be a physiologic saline buffer and may contain a detergent such as phosphate buffered saline with Tween (polyoxyethylenesorbitan). (The word TWEEN is a trade mark).

A monoclonal antibody specific for a hydrophilic epitope of the analyte is incubated with the surface for a period of time sufficient for antibody binding to the analyte. Conveniently, the incubation will be for about 30 minutes to an hour at 37° C. Following the incubation, the surface may be washed with a wash buffer to remove antibody that is not bound to the hydrophilic epitope.

Antibody bound to the hydrophilic epitope, which is bound to the surface, is detected. Typical detection means include the use of labeled antibodies, such as enzyme-linked antibodies. Similarly, the bound antibody can be detected with a labeled second antibody specific for the first antibody, Such antibodies are usually specific for the species-specific portion of the first antibody such as goat anti-mouse antibodies. The conditions of the detection step are the same as would usually be used with the detection method. That is, use of the lipophilic surface does not ater the detection steps.

The following examples are offered for purposes of clarity of understanding and not by way of limitation.

EXPERIMENTAL

Microtiter plates from Behring Diagnostics (NUNC) which represent "low", "medium", and "high" binding polystyrene were evaluated for use in the assay method. The stated binding capabilities reflect a proprietary treatment of the plastic. Aqua-Sil siliconizing solution (Pierce Chemical Co.) was used to provide a substantially uniform lipophilic surface on each type of plate. Following siliconization, each of the plates exhibited improved performance in a standardised assay, with medium binding plates (NUNC 4-69329) performing optimally in the assay procedure. Immulon 2 (Dynatech) plates were also evaluated and exhibited an enhanced binding of anti-LPS monoclonal antibodies following siliconization.
(The expression IMMULON 2 is a trade mark).

An exemplary assay for Chlamydia was performed as follows. Microtiter plates (NUNC 4-69329) were coated twice either by adding 200 μL of Aqua-Sil (Pierce Chemical Co.) siliconizing solution prepared according to the manufacturer's directions to each well or by immersing microtiter plates in a container of Aqua-Sil solution.

The sample to be treated for the presence of Chlamydia, a clinical swab containing elementary bodies (EBs), was diluted with 0.5 ml of release buffer. The release buffer formulation for the assay was as follows:

| | |
|---|---|
| 0.1M | Sodium phosphate, pH 7.4 |
| 0.088% | Sodium deoxycholate |
| 1mM | N-acetyl cysteine |
| 0.02% | Thimerosal (preservative) |

The swab and added release buffer were vortexed in a glass tube. The contents of the swab were expelled into the tube as completely as possible by pressing the swab against the wall off the tube to provide the analyte dispersed in an aqueous medium. One hundred microliters of the medium was added to pretreated microtiter wells and incubated for one hour at 37° C. Approximately 70 to 80% of available antigen binds in 1 hour at 37°C. Following incubation, sample-containing buffer was aspirated from the wells and the wells were washed once with 250 μL of PBS/Tween wash buffer (10mM Sodium Phosphate, pH 7.4 0.15M NaCl 0.1% Tween 20)

One hundred microliters of anti-Chlamydia antibody (a murine monoclonal antibody specific for the lipopolysaccharide antigen of C. trachomatis and C.psittaci diluted in 10-25% bovine calf serum, 0.025M Tris pH 8.0, 0.01M trisodium citrate, 0.02% thimerosal) was added to each well and incubated for 30 minutes at 37°C. Each well was washed twice with 250 μL of wash buffer. One hundred μL of antibody conjugate (a goat anti-mouse antibody conjugated to horseradish peroxidase enzyme and diluted in the same buffer as the anti-Chlamydia antibody) was added to each well and incubated for 30 minutes at 37°C. Each well was washed three times with wash buffer. One hundred μL of TMB enzyme substrate (Behring Diagnostics) was added per well and incubated for 30 minutes at 37°C. Following that incubation, 100 μl of stop solution (1M sulfuric acid solution) was added per well and the resultant solution was read at 450 nanometers.

It has been found using a variety of monoclonal antibodies that binding was increased by anywhere from twofold to tenfold over untreated plates.

All publications and patent applications mentioned in this specification are indicative of the level of skill of those skilled in the art to which this invention pertains.

The invention now being fully described, it will be apparent to one of ordinary skill in the art that many changes and modifications can be made thereto.

**Claims**

1. A method for assaying an amphipathic analyte comprising a hydrophilic epitope, said analyte dispersed in an aqueous medium, said method comprising:

   (a) incubating said medium with a solid substrate which has been modified to provide a substantially uniform lipophilic surface for a period of time sufficient for said amphipathic analyte to bind to said lipophilic surface;

   (b) separating said medium from said lipophilic surface; and

   (c) assaying with monoclonal antibodies for said hydrophilic epitope of said amphipathic analyte on said lipophilic surface.

2. The method of claim 1 wherein said solid substrate is modified using a siliconizing agent to provide said substantially uniform lipophilic surface.

3. The method of claim 1 wherein said solid substrate is modified by alkylation with hydrocarbon chains to provide said substantially uniform lipophilic surface.

4. A method as claimed in claim 1 for assaying a lipopolysaccharide or lipoprotein analyte in a cell-containing sample comprising a clinical swab or a tissue specimen, said method comprising:

   (a) incubating said sample with an aqueous release buffer having a total ionic strength of 0.1M to 0.5M and a pH value of from 6.5 to 8.5 and comprising 0.01mM to 0.2M sodium phosphate buffer, further comprising NaCl, 0.025 to 0.50%(w/v) detergent, and 0.01mM to 10mM reducing agent for a period of time sufficient to release lipopolysaccharide and lipoprotein from cells to provide said analyte dispersed in an aqueous medium;

   (b) incubating said medium with a substantially uniform lipophilic surface of a siliconized solid substrate for a period of time sufficient for said analyte to bind to said lipophilic surface;

   (c) separating said sample-containing medium from said lipophilic surface;

   (d) washing said lipophilic surface;

   (e) incubating a monoclonal antibody specific for a hydrophilic epitope of said analyte with said surface for a period of time sufficient for said antibody to bind to said epitope; and

   (f) detecting said antibody bound to said hydrophilic epitope of said amphipathic analyte bound to said lipophilic surface.

5. The method of claim 4 wherein said release buffer comprises 0.05M to 0.15M sodium phosphate/0.0M to 0.3M sodium chloride buffer.

6. The method of claim 4 wherein said release buffer comprises 0.05M to 0.15M sodium phosphate buffer, pH 7.4.

7. The method of claim 4 wherein said release buffer comprises 0.025 to 0.25% (w/v) of an octylglucopyranoside or deoxycholic acid derivative detergent.

8. The method of claim 7 wherein said release buffer comprises about 0.1% (w/v) deoxycholate or chenodeoxycholate detergent.

9. The method of claim 7 wherein said release buffer comprises 0.01mM to 10mM cysteine.

10. The method of any one of claims 4-9 wherein the analyte is an endotoxin diagnostic of Chlamydia.

**Patentansprüche**

1. Verfahren zur Bestimmung einer amphipatischen Substanz, die ein hydrophiles Epitop aufweist, wobei die Substanz in einem wässerigen Medium dispergiert ist und wobei das Verfahren folgendes umfaßt:

 (a) Inkubieren des Mediums mit einem festen Substrat, das zur Bildung einer im wesentlichen gleichförmigen lipophilen Oberfläche verändert wurde, über einen Zeitraum, der zur Bindung der amphipatischen Substanz an die lipophile Oberfläche ausreicht;

 (b) Trennen des Mediums von der lipophilen Oberfläche; und

 (c) Bestimmen des hydrophilen Epitops der amphipatischen Substanz an der lipophilen Oberfläche mit monoklonalen Antikörpern.

2. Verfahren nach Anspruch 1, worin das feste Substrat durch Verwendung eines Silikonisierungsmittels verändert wird, um die im wesentlichen gleichförmige lipophile Oberfläche zu erhalten.

3. Verfahren nach Anspruch 1, worin das feste Substrat durch Alkylierung mit Kohlenwasserstoffketten verändert wird, um die im wesentlichen gleichförmige lipophile Oberfläche zu erhalten.

4. Verfahren nach Anspruch 1 zur Bestimmung einer Lipopolysaccharid- oder Lipoproteinsubstanz in einer zellhaltigen Probe, bestehend aus einem Klinischen Abstrich oder einer Gewebsprobe, wobei das Verfahren folgendes umfaßt:

 (a) Inkubieren der Probe mit einem wässerigen Trennpuffer mit einer Gesamtionenstärke von 0,1M bis 0,5M und einem pH-Wert von 6,5 bis 8,5 und bestehend aus 0,01mM bis 0,2M Natriumphosphatpuffer, und ferner bestehend aus NaCl, 0,025 bis 0,50 (Gew.-/Vol.-%) Detergens und 0,01mM bis 10mM Reduktionsmittel über einen Zeitraum, der für die Trennung des Lipopolysaccharids und Lipoproteins von Zellen ausreicht, so daß eine Dispersion der Substanz in einem wässerigen Medium erhalten wird;

 (b) Inkubieren des Mediums mit einer im wesentlichen gleichförmigen lipophilen Oberfläche eines silikonisierten festen Substrats über einen Zeitraum, der für die Bindung der Substanz an der lipophilen Oberfläche ausreicht;

 (c) Trennen des probenhaltigen Mediums von der lipophilen Oberfläche;

 (d) Waschen der lipophilen Oberfläche;

 (e) Inkubieren eines monoklonalen Antikörpers, der für ein hydrophiles Epitop der Substanz spezifisch ist, mit der Oberfläche über einen Zeitraum, der für die Bindung des Antikörpers an das Epitop ausreicht; und

 (f) Nachweisen des Antikörpers, der an das hydrophile Epitop der amphipatischen Substanz gebunden ist, das an die lipophile Oberfläche gebunden ist.

5. Verfahren nach Anspruch 4, worin der Trennpuffer 0,05M bis 0,15M Natriumphosphat/0,0M bis 0,3M Natriumchloridpuffer umfaßt.

6. Verfahren nach Anspruch 4, worin der Trennpuffer 0,05M bis 0,15M Natriumphosphatpuffer, pH 7,4, umfaßt.

7. Verfahren nach Anspruch 4, worin der Trennpuffer 0,025 bis 0,25 (Gew.-/Vol.-%) eines Octylglucopyranosid- oder Desoxycholsäurederivatdetergens umfaßt.

8. Verfahren nach Anspruch 7, worin der Trennpuffer etwa 0,1 (Gew.-/Vol.-%) Desoxycholat- oder Chenodesoxycholatdetergens umfaßt.

9. Verfahren nach Anspruch 7, worin der Trennpuffer 0,01mM bis 10mM Cystein umfaßt.

10. Verfahren nach einem der Ansprüche 4 bis 9, worin das Analyt ein Endotoxin ist, das für Chlamydia kennzeichnend ist.

**Revendications**

1. Procédé pour doser une espèce à analyser amphipathique comprenant un épitope hydrophile, l'espèce à analyser étant dispersée dans un milieu aqueux, le procédé comprenant :

 (a) l'incubation du milieu avec un substrat solide qui a été modifié pour présenter une surface lipophile sensiblement uniforme pendant un temps suffisant pour que l'espèce à analyser amphipathique se lie à la surface lipophile;

 (b) la séparation entre le milieu et la surface lipophile;

 (c) le dosage, à l'aide d'anticorps monoclonaux, de l'épitope hydrophile de l'espèce à analyser amphipathique sur la surface lipophile.

2. Procédé suivant la revendication 1, dans lequel le substrat solide est modifié au moyen d'un agent de siliconation pour former la surface lipophile sensiblement uniforme.

3. Procédé suivant la revendication 1, dans lequel le substrat solide est modifié par alcoylation au moyen de chaînes hydrocarbonées pour former la surface lipophile sensiblement uniforme.

4. Procédé suivant la revendication 1, pour doser un lipopolysaccharide ou une lipoprotéine comme espèce à analyser dans un échantillon contenant des cellules comprenant un écouvillon clinique ou un prélèvement de tissu, le procédé comprenant :

(a) l'incubation de l'échantillon avec un tampon aqueux de libération ayant une force ionique totale de 0,1 M à 0,5 M et un pH de 6,5 à 8,5 comprenant un tampon de phosphate de sodium 0,01 mM à 0,2 M et comprenant aussi du NaCl, 0,025 à 0,50% (p/v) de détergent et un agent réducteur 0,01 mM à 10 mM, pendant un temps suffisant pour libérer le lipopolysaccharide et la lipoprotéine des cellules afin de former l'espèce à analyser dispersée dans un milieu aqueux;

(b) l'incubation du milieu avec une surface lipophile sensiblement uniforme d'un substrat solide siliconé pendant un temps suffisant pour que l'espèce à analyser se lie à la surface lipophile;

(c) la séparation entre le milieu contenant l'échantillon et la surface lipophile;

(d) le lavage de la surface lipophile;

(e) l'incubation d'un anticorps monoclonal spécifique pour un épitope hydrophile de l'espèce à analyser avec la surface pendant un temps suffisant pour que l'anticorps se lie à l'épitope, et

(f) la détection de l'anticorps lié à l'épitope hydrophile de l'espèce à analyser amphipathique liée à la surface hydrophile.

5. Procédé suivant la revendication 4, dans lequel le tampon de libération comprenant un tampon de phosphate de sodium 0,05 M à 0,15 M/chlorure de sodium 0,0 M à 0,3 M.

6. Procédé suivant la revendication 4, dans lequel le tampon de libération comprend un tampon de phosphate de sodium 0,05 M à 0,15 M de pH 7,4.

7. Procédé suivant la revendication 4, dans lequel le tampon de libération comprend 0,025 à 0,25% (p/v) d'un octylglucopyrannoside ou dérivé d'acide désoxycholique détergent.

8. Procédé suivant la revendication 7, dans lequel le tampon de libération comprend environ 0,1% (p/v) de désoxycholate ou chénodésoxycholate détergent.

9. Procédé suivant la revendication 7, dans lequel le tampon de libération comprend de la cystéine 0,01 M à 10 mM.

10. Procédé suivant l'une quelconque des revendications 4 à 9, dans lequel l'espèce à analyser est une endotoxine diagnostique de Chlamydia.

Fig. I
No Siliconization

Solid phase (2)

Aqueous phase (7)

Lipid (1) containing molecules

Random Orientation

Fig. II
with Siliconization

Solid phase (2)

Aqueous (7) phase

Lipid (1) containing molecules

Siliconizing (3) Layer

Polar / Non Polar Orientation

EP 0 303 515 B1